(19) Europäisches Patentamt
European Patent Office
Office européen des brevets

(11) **EP 4 563 558 A1**

(12) **EUROPEAN PATENT APPLICATION**
published in accordance with Art. 153(4) EPC

(43) Date of publication:
**04.06.2025 Bulletin 2025/23**

(21) Application number: **23845757.6**

(22) Date of filing: **14.07.2023**

(51) International Patent Classification (IPC):
***C07B 43/04*** *(2006.01)* ***C07C 209/60*** *(2006.01)*

(52) Cooperative Patent Classification (CPC):
**C07B 43/04; C07C 209/60**

(86) International application number:
**PCT/ES2023/070456**

(87) International publication number:
**WO 2024/023383 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.07.2022 ES 202230686**

(71) Applicants:
- **CONSEJO SUPERIOR DE INVESTIGACIONES CIENTIFICAS (CSIC)**
**28006 Madrid (ES)**
- **Universitat Politècnica de València**
**46022 Valencia (ES)**

(72) Inventors:
- **PRIETO GONZÁLEZ, Gonzalo**
**46022 Valencia (ES)**
- **CETERONI, Ilaria**
**46022 Valencia (ES)**
- **LLOPIS PÉREZ, Sebastián**
**46022 Valencia (ES)**

(74) Representative: **Pons**
**Glorieta Rubén Darío 4**
**28010 Madrid (ES)**

# (54) METHOD FOR PRODUCING AMINES

(57) The invention discloses a method for producing alkylamines using synthesis gas reaction and a nitrogen compound over a solid catalyst in a single reaction stage. The method results in high alkylamine selectivity with low carbon dioxide and non-nitrogen hydrocarbon production. The invention further discloses a method in which the produced alkylamines are mainly fatty amines with hydrocarbon chains having between 7 and 30 carbon atoms. The invention also discloses a solid catalyst for said method.



Processed by Luminess, 75001 PARIS (FR)

## Description

### FIELD OF THE INVENTION

The invention relates to a method for producing alkylamines, including fatty amines, by reacting synthesis gas and a nitrogen-containing compound over a solid catalyst.

### BACKGROUND

Alkylamines are chemical compounds with a general molecular formula

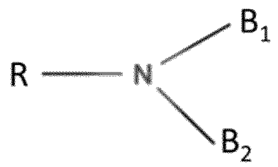

in other words, R-N-[$B_1$,$B_2$], wherein the length of the carbyl substituents R, $B_1$ and $B_2$ attached to the central N atom determines their technical properties and fields of application. In a prevalent class of alkylamines, the longest substituent R is an alkyl group which typically consists of 4 or more carbon atoms and defines the molecular backbone of the alkylamine compound, while substituents $B_1$ and $B_2$ are either H atoms, shorter alkyl substituents, with 1-3 carbon atoms, or two ends of a cycloalkyl group. Therefore, alkylamines are classified as primary, secondary and tertiary amines depending on whether both, only one or none of substituents $B_1$ and $B_2$ are H atoms, respectively.

Fatty amines, wherein R is a $C_n$ alkyl substituent, typically with n between 7 and 30 carbon atoms, and their derivatives, for example, quaternary ammonium compounds, find applications as surfactants, emulsifiers, flotation agents and anti-caking agents in different industries, such as the paint and coating, textile, personal care, extractive mining and the oil & gas industry. Tertiary fatty amines, wherein R is a $C_n$ alkyl substituent with n=7-30, and $B_1$ and $B_2$ are $C_m$ alkyl substituents, typically with m≤3, for example, methyl ($C_1$), ethyl ($C_2$), isopropyl ($C_3$), etc., are interesting as preservative components, fuel oils additives, fungicides, rare metal extractants , cosmetics, active ingredients in polymer manufacture , accelerators and coagulants for foams and resins, as well as cationic, non-ionic and amphoteric surfactant precursors, among others. Short-chain alkylamines, wherein R is a $C_n$ alkyl substituent with n=4-6, find applications as precursors for pharmaceutical products, pesticides, dyes, solvents, chromatography agents in life-sciences, rubber vulcanisation accelerators, and plasticisers, among others.

Alkylamines are usually produced by amination or reductive amination of pre-existing alkyl compounds, such as aldehydes, alcohols, olefins or aliphatic organohalide compounds, with nitrogen-containing compounds (N-compounds), such as amines or ammonia. Alternatively, alkylamines can also be produced via hydrogenation of aliphatic nitriles. The document A. Baiker and J. Kijenski, Catalytic Synthesis of Higher Aliphatic Amines from the Corresponding Alcohols, Catal. Rev. -Sci. Eng. 27(4), 653-697 (1985) provides an overview of existing methods for producing alkylamines.

Alternatively, alkylamines can be produced by hydroamination, that is the catalytic addition of an N-H bond to an unsaturated C-C bond of a pre-existing aliphatic olefin, as described, for example, in documents US 7,642,383 B2, US 4,138,437, US 5,043,453A.

Another method for the synthesis of alkylamines is the so-called hydro-aminomethylation of olefins, in which an aliphatic olefin is reacted with carbon monoxide, hydrogen and a nitrogen-containing compound, such as an amine or ammonia, to produce alkylamines with a hydrocarbon chain length extended in one carbon atom with respect to the starting olefin. The reaction was first reported by Reppe and Vetter using a molecular cobalt carbonyl catalyst (Reppe, W., & Vetter, H. (1953), "Carbonylierung VI. Synthesen mit Metallcarbonylwasserstoffen". Justus Liebigs Annalen der Chemie, volume 582, 133-161). Document US4250115A refers to a method for the preparation of tertiary amines comprising the reaction of an olefin compound, carbon monoxide, hydrogen and a nitrogen-containing compound of formula H-N-$R_2$, wherein R is selected from the group consisting of hydrogen radicals, alkyl radicals, aryl, cycloalkyl, arylalkyl, and alkyl in the presence of a rhodium catalyst. Document US4543411A relates to a method for preparing secondary and tertiary amines which comprises reacting an olefin, a nitrogen-containing compound and synthesis gas in the presence of a catalyst comprising a ruthenium-containing compound combined with an organic ligand consisting of a quaternary onium salt. Document US4096150A refers to a method for preparing substituted amines with high selectivity by reacting an olefin reagent, hydrogen, CO, and secondary amine in the presence of a coordination complex catalyst of a group VIII metal and a ligand. Documents WO2005077884A2 and DE10321421A1 also disclose methods for the synthesis of alkylamines by means of hydro-aminomethylation of olefins.

Common to the above methods to prepare alkylamines is that the alkyl hydrocarbon substituent (R=Cn) in the alkylamine end product needs to be provided in the form of a constituent part (synthon) of a reactant compound, for

example, an aldehyde, an alcohol, an olefin, or an alkyl halide. In the case of methods of hydro-aminomethylation of olefins, the alkyl chain of a $C_{n-1}$ olefin reactant is extended in one single carbon atom to form the substituent R ($C_n$) in the alkylamine product. In methods which target the production of fatty amines, the reactant providing the alkyl substituent (R) may be a natural fatty acid or a derivative thereof. Although these methods of the art are effective to selectively produce alkylamines, they require the supply of a pre-formed $C_n$ or $C_{n-1}$ group as part of one of the reactants of the method. This reactant needs to be produced in a previous and separate method, or to be isolated from natural sources. Therefore, it is desirable to develop methods for the selective production of alkylamines in which more versatile and cost-effective monocarbonated compounds ($C_1$) are fed, as the only precursors of the hydrocarbon backbone in the alkylamine products.

Synthesis gas, commonly abbreviated as "syngas", is a mixture composed of carbon monoxide (CO), hydrogen ($H_2$) and, in some cases, also carbon dioxide ($CO_2$) as the main components. Synthesis gas streams may also include other gases such as nitrogen ($N_2$), helium (He), argon (Ar), water vapour ($H_2O$) or light hydrocarbons such as methane ($CH_4$), ethane ($C_2H_6$), propane ($C_3H_8$), which do not significantly alter the reactivity of the major components. Synthesis gas can be obtained from a wide array of carbonaceous sources, for example, by means of steam reforming or partial oxidation of natural gas or shale gas, gasification of coal, gasification and/or reforming of biomass, hydrogenation of carbon dioxide or co-electrolysis of $CO_2$ and water, among others, so it is considered a very versatile $C_1$ raw material for the production of chemical products from a wide array of carbonaceous feedstocks alternative to petroleum.

Fischer-Tropsch synthesis is a catalytic reaction whereby synthesis gas is directly converted to a mixture of synthetic aliphatic hydrocarbons, mainly n-paraffins (Fischer-Tropsch Synthesis, Catalysts, and Catalysis: Advances and Applications; B. H. Davis, M. L. Occelli (Eds), CRC Press (2016), ISBN 978-1466555297). Earlier work is known to those skilled in the art on processes wherein a synthesis gas feed is mixed with a gaseous nitrogen source, such as ammonia, and said gas mixture is contacted with a catalyst, under standard conditions for Fischer-Tropsch synthesis, to promote the formation of N-compounds. Normally, a complex mixture of N-compounds is produced along with non-nitrogenated hydrocarbons.

Document US 2,518,754 discloses a method for the synthesis of aliphatic amines which comprises contacting a gaseous mixture containing synthesis gas and ammonia with an iron-containing hydrogenation catalyst and recovering aliphatic amines from the products of said reaction. The condensed reaction products consist of a mixture of primary aliphatic amines and nitrogen-free hydrocarbons, as well as ammonium carbonate salt.

Document US 2,821,537 discloses a method for the catalytic hydrogenation of carbon monoxide with the production of primary synthesis products containing nitrogen-containing compounds in addition to a relatively high content of oxygen-containing compounds, preferably alcohols. A syngas mixture, together with 0.5 to 2% by volume of a nitrogen-containing compound selected from the group consisting of ammonia and methylamine, is fed, and the mixture reacts on a precipitated iron catalyst. The reaction products contain N-compounds, including amines but, furthermore, more than 20% of oxygen-containing compounds, and a high content of non-nitrogenated olefinic hydrocarbons.

Document US 3,726,926 discloses a method for preparing n-alkylamines by contacting a gaseous feed consisting of a mixture of synthesis gas and ammonia with an iron-based catalyst and recovering n-alkylamines from the reaction products. Under the method conditions, the organic products comprise a mixture of non-nitrogenated hydrocarbons and aliphatic amines with $C_3$ to $C_{22}$ alkyl chains. Due to the side-production of non-nitrogenated hydrocarbons, the highest selectivity to amines described in the document is 35%.

Document WO2009/127942A2 discloses a method for producing at least one nitrogen- or phosphorus-containing compound selected from linear nitriles, amides, formamides, and phosphorus-containing compounds from synthesis gas, characterised in that the synthesis gas, together with at least one nitrogen- or phosphorus-containing compound, are fed into a reactor and react over a catalyst, preferably consisting of unsupported and promoted iron. The method is carried out at temperatures comprised between 433 K and 673 K and at pressures between 1 and 50 bar. Ammonia, nitrogen oxides $NO_x$ ($N_2O$, NO, $NO_2$) and mixtures thereof are disclosed in the document as nitrogen-containing compounds that can be fed, as a source of nitrogen, and react together with the synthesis gas. Therefore, both the synthesis gas components and the nitrogen sourcing compounds are gaseous under the method conditions. The organic products comprise a mixture of non-nitrogenated hydrocarbons (paraffins, olefins and oxygenates) and nitrogenated organic compounds. Along with linear nitriles, amides, or amide-formamide compounds targeted by the method, amines are also produced. However, as a result of the concomitant production of oxygenates and nitrogen-free hydrocarbons, amine selectivity is limited to $\leq$6.6% under the conditions disclosed in the document. As disclosed in T. Sango, et al. Applied Catalysis A: General 502 (2015) 150-156 by the same authors, the amine compounds produced are primary n-alkylamines, with R alkyl substituent chains that are mostly between 2 and 13 carbon atoms in length. It should be noted that a significant amount of $CO_2$ is also produced under the method conditions, with a $CO_2$ selectivity that varies between 35 and 40% (carbon basis).

Document WO225870 A1 discloses a hydrogenation method for producing N-compounds which consists of contacting a gaseous feed stream, consisting of a mixture of synthesis gas and ammonia, with a solid catalyst, preferably comprising cobalt and manganese, to form at least one aliphatic amine and/or one nitrile compound. Under preferred

method conditions, a maximum selectivity to aliphatic amines of 25% is disclosed in said document.

There is a need for a method to directly produce alkylamines from synthesis gas and nitrogen-sourcing compounds, with greater selectivity, in other words, by minimising or directly avoiding the co-production of lower value side-products. One of the main limitations of the methods known in the art is the co-production of non-nitrogenated compounds, in other words, paraffins, olefins and oxygenated compounds. Without wishing to be bounded by theory, the inventors have considered that this is due to an insufficient rate of amination reactions on the surface of the catalyst. Higher selectivity to alkylamines would translate into lower product separation and purification costs, as well as a reduced side-production of waste or low value-added products and, therefore improved process efficiency and economics.

Therefore, one of the objectives of the present invention is to provide a method for producing alkylamines with high selectivity, and minimum side-production of nitrogen-free hydrocarbons and $CO_2$ by reacting synthesis gas , as the sole carbon source for the R alkyl chain in the amine products, and a nitrogen-sourcing reactant as the sole nitrogen source in the amine products, over a solid catalyst, in a single reaction step. It is an additional objective of the present invention to provide a method in which the alkylamine products are fatty amines. It is yet an additional objective of the present invention to provide a solid catalyst for said method.

## DESCRIPTION

It has been found that said method can be implemented by means of at least the following steps:

i) providing a reactor with

a. a synthesis gas feed, and

b. a liquid feed comprising a nitrogen-sourcing reactant selected from the list of primary amine compounds, secondary amines, acyclic secondary amines, cyclic secondary amines including non-N-substituted compounds piperidine, morpholine, pyrrolidine, pyrrole, imidazole, N,N-dialkylformamide compounds, N,N-cycloalkylformamide compounds, and any combination thereof, which can be either in neat form, or dissolved in a suitable solvent which does not react under the method conditions,

ii) reacting the feed provided in i) over a solid catalyst comprising at least ruthenium, cobalt, or any combination thereof, preferably ruthenium, either in unsupported form or dispersed on a support material, at a reaction temperature comprised between 373 and 523 degrees Kelvin (K) and a total pressure comprised between 1 bar and 300 bar; and

iii) recovering alkylamine compounds from the products of said reaction.

As mentioned above, the present invention relates, in a first aspect to a method for producing alkylamines. The present invention relates, in a second aspect, to the method wherein the alkylamine products are fatty amines. The present invention relates, in a third aspect, to a solid catalyst for said method.

The term "nitrogen-sourcing reactant", as it is used in the context of the present invention, is understood to mean a nitrogen-containing compound, which participates and it is consumed in the method, and it sources reactive nitrogen species.

The term "conversion", as it is used in the context of the present invention, is expressed as a percentage and is understood to mean the fraction of $CO_x$ (x=1 or 2), in the synthesis gas feed, which is converted in the reactor into other carbon-containing compounds.

The term "selectivity", as it is used in the context of the present invention, is defined on a carbon basis and, therefore, is understood to mean the ratio of the total molar amount of carbon in the products of interest, for example, alkylamines, and the total molar amount of carbon in all reaction products.

### *Method*

According to the method of the present invention, the catalyst is contacted with at least one liquid phase, which remains liquid under the method conditions. Therefore, reactor types which are suitable for implementing the method of the invention are those which enable contacting the gas and liquid phases with a solid catalyst.

In one embodiment of the invention, the reactor is operated in a continuous mode, whereby at least one stream of the gas feed and one stream of the liquid feed are provided continuously to the inlet of the reactor, for example, a bubble column reactor or a trickle bed reactor, and a product stream is continuously removed at the outlet of the reactor.

In another embodiment of the invention, the reactor is operated in a discontinuous mode (batch type), whereby gaseous and liquid feeds, as well as the catalyst, are provided in the reactor before the reactor temperature is adjusted to the reaction temperature, the reaction proceeds without additional streams being provided at the inlet of the reactor during

the reaction. After a certain reaction time, the reaction is terminated by lowering the reactor temperature below the temperature range for the method of the invention, and at least one product stream is recovered from the reactor.

In another embodiment of the invention, the reactor is operated in a semi-continuous mode, whereby gaseous and liquid feeds, as well as the catalyst, are provided in the reactor before the reactor temperature is adjusted to the reaction temperature, the reaction proceeds under continuous supply of a synthesis gas feed stream at the inlet of the reactor, the reaction is terminated after a certain reaction time by lowering the reactor temperature below the temperature range for the method of the invention, and at least one product stream is recovered from the reactor.

According to the present invention, the $H_2/CO$ molar ratio in the synthesis gas feed can be in the range of 0.1 to 10, preferably said $H_2/CO$ molar ratio in the synthesis gas is in the range of 0.3 to 5, and more preferably in the range of 0.5 to 2.

According to the present invention, the $CO_2/CO$ molar ratio in the fed synthesis gas can be in the range of 0 to 2. In a preferred embodiment of the present invention, said $CO_2/CO$ molar ratio in the synthesis gas is in the range of 0 to 0.5. More preferably, the $CO_2/CO$ molar ratio in the synthesis gas is in the range of 0 to 0.1.

The synthesis gas feed may further comprise other compounds that do not interfere with synthesis gas conversion, such as nitrogen, helium, argon, water, $C_1$-$C_4$ alkanes, or combinations thereof.

The liquid feed to the method of the present invention contains a nitrogen-sourcing reactant. Without wishing to be bounded by theory, the inventors consider that compounds suitable as nitrogen-sourcing reactants are nitrogen compounds having at least one N-H bond which can participate in hydroamination or reductive amination reactions. Alternatively, compounds suitable as a nitrogen-sourcing reactant are compounds which can react under the conditions of the method of the invention, generating compounds having at least one N-H bond. According to one embodiment of the invention, the nitrogen-sourcing reactant can be selected from the group of primary amine compounds, such as, for example, methylamine, ethylamine, propylamine, isopropylamine; acyclic secondary amines, such as dimethylamine, diethylamine, dipropylamine, diisopropylamine, N-ethylmethylamine, N-methylpropylamine, etc.; cyclic secondary amines including non-N-substituted compounds piperidine, morpholine, pyrrolidine, pyrrole, imidazole; N,N-dialkylformamide compounds, such as N,N-dimethylformamide, N,N-diethylformamide, N-methyl,N-ethylformamide, N,N-diisopropylformamide, etc.; N,N-cycloalkylformamide compounds, such as piperidinoformamide (piperidine-1-carbaldehyde), prolinal (pyrrolidine-1-carbaldehyde), etc.; non-N-substituted compounds of piperidine, morpholine, pyrrolidine, pyrrole, imidazole type, and any combination thereof.

According to a preferred embodiment of the invention, said nitrogen-supplying reagent is N,N-dialkylformamide, and more preferably N,N-dimethylformamide.

According to one embodiment of the invention, the nitrogen-sourcing reactant can be supplied as a neat compound or as part of a solution, in other words, dissolved in a suitable solvent that does not react and is stable under the method conditions. The solvent used in the method of the present invention is not particularly limited, provided that the nitrogen-sourcing reactant shows at least partial solubility in said solvent and the solvent remains at least partially as a liquid under the conditions of the method of the invention. The solvent can be selected from the list of aliphatic hydrocarbons, such as n-alkanes, selected from the non-limiting list of n-hexane, n-heptane, n-octane, n-decane, n-dodecane, n-hexadecane, waxes, or combinations thereof, or isoalkanes selected from the non-limiting list of isohexane, isooctane, isohexadecane, or combinations thereof, or cyclic alkanes such as cyclohexane; aromatic hydrocarbons, selected from the non-limiting list of benzene, toluene, xylenes, ethylbenzene, dimethylbenzenes, trimethylbenzenes, and combinations thereof; diesel; oxygenated compounds, selected from the non-limiting list of n-butanol, isobutanol, cyclohexanol, tetrahydrofuran, methylisobutylketone, ethylene glycol, propylene glycol, glycerol, and combinations thereof; water, nitrogen compounds, selected from the non-limiting list of tertiary aliphatic amines, N-methylmorpholine, N-methylpiperidine, N-methylpyrrole, aromatic amines, and combinations thereof; halogenated organic compounds, selected from the non-limiting list of chlorobenzenes, bromobenzenes, hexachlorocyclopentadiene, perfluorodecalin, and combinations thereof; liquid organic salts selected from the non-limiting list of imidazolium, pyridinium, 1,2,3-triazolium, piperidinium, phosphonium, and combinations thereof; as well as combinations thereof.

According to another embodiment of the invention, a single N-compound is fed into the reactor to act both as a nitrogen-sourcing reactant and a solvent which provides the liquid medium under the method conditions. Without wishing to be bounded by theory, the inventors believe that a high molar content of the nitrogen-sourcing reactant in contact with the solid catalyst, can provide excess of activated nitrogen species on the surface of the catalyst and, thus, contribute to a higher reaction rate for the amination reactions of aliphatic Fischer-Tropsch intermediates compared to competing hydrogenation reactions which are responsible for the production of non-nitrogenated paraffins. This high molar content of the nitrogen-sourcing reactant in contact with the solid catalyst can be achieved when the catalyst works in contact with a liquid solution of the nitrogen-sourcing reactant, or directly with nitrogen-sourcing reactant in neat form. Furthermore, the application of a single compound acting both as a nitrogen-sourcing reactant and a solvent facilitates method design and product recovery, since the reaction products can be separated from the solvent, and the latter can be recycled into the reactor, with a minor makeup addition to compensate for its consumption in the reaction. In a preferred embodiment of the invention, N,N-dimethylformamide is fed into the reactor to act both as a nitrogen-sourcing reactant and a solvent.

According to the present invention, the method is carried out at a reaction temperature in the range from 373

degrees Kelvin (K) to 523 K. The reaction temperature, as it is used in the context of the present invention, is understood to mean the maximum temperature in the reactor measured by means of a K-type thermocouple located inside a stainless-steel sheath and in contact with the liquid phase. In a preferred embodiment of the present invention, the method can be carried out at a reaction temperature in the range from 393 K to 453 K, and more preferably in the range from 413 K to 443 K.

Suitable reaction pressures for the method of the invention are from about 1 bar to about 300 bar. Operating pressure, as it is used in the context of the present invention, is understood to mean the absolute pressure in the reactor headspace occupied by the gas phase, measured by means of a digital pressure transducer under the method conditions. In the case of batch reactor operations, in which pressure decreases in the course of the synthesis gas conversion process, the reaction pressure, as it is used in the context of the present invention, is understood to mean the pressure measured at the beginning of the reaction. In a preferred embodiment, the method can be carried out at a reaction pressure in the range from 10 bar to 300 bar, and more preferably in the range from 40 bar to 200 bar.

The nature of the nitrogen-sourcing reactant fed to the method of the invention predominantly determines the type of alkylamine products. Therefore, if the nitrogen-sourcing reactant is a primary amine of formula $B_1$-N-$H_2$, wherein $B_1$ is an alkyl substituent, the amine products consist mainly of secondary alkylamines of formula [R,$B_1$]-N-H, wherein R is an alkyl substituent with n carbon atoms (n=4-60). Moreover, if the nitrogen-sourcing reactant is a secondary amine of formula [$B_1$,$B_2$]-N-H, or N,N-dialkylformamide of formula [$B_1$,$B_2$]-NCHO, wherein $B_1$ and $B_2$ are alkyl or cycloalkyl substituents at the N atom, the amine products consist of tertiary alkylamines of formula R-N-[$B_1$,$B_2$], wherein R is an alkyl substituent with n carbon atoms (n=4-60). In a preferred embodiment of the present invention, the main products are tertiary alkylamines with formula R-N-[$B_1$,$B_2$], wherein R is an alkyl substituent with n carbon atoms (n=4-60).

According to a particular embodiment, alkylamine products are tertiary fatty amines with molecular formula R-N-($B_1$,$B_2$), wherein the substituent group R is an n-alkyl group having between 7 and 30 carbon atoms, and wherein the substituent groups $B_1$ and $B_2$ are selected from the list of methyl, ethyl, propyl, or isopropyl, and more preferably the substituent groups $B_1$ and $B_2$ are methyl groups.

According to another particular embodiment, alkylamine products are tertiary fatty amines with molecular formula R-N-($B_1$,$B_2$), wherein the substituent group R is an n-alkyl group having between 7 and 30 carbon atoms, and wherein the substituent groups $B_1$ and $B_2$ are the two ends of a ring having four carbon atoms or a ring having five carbon atoms.

In the method of the invention, the alkylamine product distribution typically follows an Anderson-Schulz-Flory (ASF) type chain length distribution, which is typically observed for reactions proceeding via a chain-growth polymerisation mechanism. This product distribution is mathematically described by means of the following relationship (equation 1):

$$W_n = n \cdot (1-\alpha)^2 \cdot \alpha^{n-1} \qquad \text{(equation 1)}$$

where $W_n$ is the mass fraction of alkylamines for which the longest $C_n$ alkyl substituent bonded to the N atom contains n carbon atoms, and $\alpha$ (alpha) is the chain growth probability parameter, which represents the probability, on a 0 to 1 basis, that a $C_n$ hydrocarbon chain undergoes the addition of a monomeric species, and thus grows by one carbon atom, under the reaction conditions. Figure 1 shows the evolution of the relative abundance of products with alkyl chain lengths in the range of $C_{7-30}$, that is, corresponding to the fatty alkylamine range, as a function of alpha, according to the product distribution predicted by equation 1. As observed, alpha values between 0.80 and 0.90 maximise the relative contribution of products with alkyl chain lengths in the fatty amine range. Experimentally, the chain growth probability parameter (alpha) can be determined according to equation 2:

$$\alpha = e^m \qquad \text{(equation 2)}$$

where e is the Euler's number and m represents the slope obtained for the mathematical linear regression function of the experimental data of the reaction products on a graph plotting Ln($W_n$/n) versus n over a sufficiently broad range of carbon chain lengths n, wherein Ln represents the natural logarithm function.

Typically, the product mixture of the method of the invention is fractionated to recover the amine products. In a particular embodiment of the invention, after recovering alkylamines from the reaction products, compounds present in the reactor effluent stream, such as reagents that have not been converted, solvent, reaction products, or a combination thereof, are recovered and circulated back to the reactor.

### *Catalyst*

According to the present invention, the catalyst used in the method according to step ii) is a solid comprising at least ruthenium (Ru), cobalt (Co), or any combination thereof, preferably ruthenium, as an active metal, which may be in unsupported form or dispersed on a support material.

In a particular embodiment of the invention, the catalyst is an unsupported metal catalyst (bulk type), such as a finely divided metal powder (metal black), a metal gauze, or a metal foam.

In another particular embodiment of the invention, the catalyst is a supported metal catalyst. The concept of "supported metal catalyst" is understood in the art as a composition which comprises the active metal part, dispersed in the form of particles with a mean diameter typically smaller than 100 nm, which is therefore active or can be converted into an active phase *in situ* before or during the use of the catalyst, and a catalytically non-active part, in which the catalytically non-active part, denoted as carrier or support material, is generally porous and forms most of the catalyst on a mass basis.

In a particular embodiment of the invention, the active metal also comprises, together with Ru, Co, or combinations thereof, other elements selected from the list of rhodium (Rh), iridium (Ir), or any combination thereof. According to a preferred embodiment of the invention, Ru represents more than 25% by weight of the active metal. More preferably, Ru represents more than 50% by weight of the active metal.

Several methods are known in the art for incorporating the active metal part on a support material. These methods comprise preparation techniques such as impregnation, precipitation-deposition, ion exchange, electrochemical deposition, electrostatic adsorption, melt infiltration, coprecipitation, which are methods known in the art. The documents "Handbook of Heterogeneous Catalysis", G. Ertl, H. Knözinger, F. Schüth, J. Weitkamp (Editors); Volume 1, Wiley-VCH Verlag GmbH & Co. Weinheim, 2008 and "Synthesis of solid catalysts", K. P. de Jong (Editor); Wiley-VCH Verlag GmbH & Co. Weinheim, 2009 provide references on existing methods for incorporating the active metal part on a support material.

The porous support material should be chemically stable under the reaction conditions typical of a synthesis gas conversion process. Said porous support can be an oxide selected from those porous oxide supports that are used as support materials for supported metal catalysts in the art, and may comprise, but is not limited to, $SiO_2$, transition forms of $Al_2O_3$, $\alpha$-$Al_2O_3$, $TiO_2$, or combinations thereof. Alternatively, the support material can be a carbide or oxycarbide material such as SiC, $SiO_xC_y$, with x being in the range $0<x<2$ and y being in the range $0<y<1$. Alternatively, the support material can be carbon. Alternatively, the support material can be a composite incorporating a combination of said materials.

According to a preferred embodiment, the porous support is $Al_2O_3$.

Metals are incorporated on the support material using metal precursor compounds such as metal inorganic salts, selected from the non-limiting list of nitrates, halides, carbonates, sulphates, and combinations thereof, organic salts selected from the non-limiting list of acetates, acetylacetonates, oxalates, ethoxylates, and combinations thereof, metal clusters and organometallic complexes.

Some specific examples of ruthenium-containing precursors are ruthenium acetylacetonate, ruthenium chloride, ruthenium nitrosyl nitrate, triruthenium dodecacarbonyl, bis(cyclopentadienyl)ruthenium(II), and the like. Ruthenium precursors can provide ruthenium in a zero oxidation state, oxidation state II, oxidation state III, or a combination thereof. According to a preferred embodiment of the invention, the catalyst is prepared using ruthenium nitrosyl nitrate as ruthenium precursor. Some specific examples of cobalt-containing precursors are cobalt nitrate, cobalt acetylacetonate, cobalt acetate, cobalt oxalate, cobalt chloride, dicobalt octacarbonyl, and the like. Cobalt precursors can provide cobalt in a zero oxidation state, oxidation state II, oxidation state III, or a combination thereof.

According to a preferred embodiment of the invention, the catalyst is prepared using cobalt nitrate as the cobalt precursor.

Typically, after incorporating the metal precursor, a thermal treatment is applied under either a stagnant gas atmosphere or flowing air, steam, an inert gas such as nitrogen, helium, argon, or combinations thereof, to decompose the metal precursor into an oxide or suboxide form on the surface of the support material.

Preferably, the weight loading of the active metal, in other words, the weight fraction of the total catalyst corresponding to the active metal in said catalyst, is in the range of 0.5-50% by weight, and more preferably in the range of 3-20% by weight.

It is common in the art that supported metal catalysts comprise so-called promoters, in addition to the main active metal part and the support material. Generally, a promoter is a substance that improves the performance of the catalyst in terms of activity, selectivity with respect to a given product or product fraction, stability under operating conditions, or combinations thereof. In one embodiment of the invention, the catalyst comprises at least one promoter. Said promoter may contribute to a higher alkylamine product selectivity under the conditions of the method of the present invention. Without wishing to be bounded by theory, the inventors believe that a suitable promoter can facilitate the activation of the nitrogen-sourcing reactant for its participation in amination reactions with the intermediates from synthesis gas conversion on the surface of the catalyst.

According to a particular embodiment, said promoter can be selected from the list of elements from groups 3, 4, 5, 6, 7, 12, 13, 14, 15, and lanthanides of the periodic table, and any combination thereof. In a preferred embodiment of the invention, said promoter is selected from the list of Y, Ti, Zr, V, Nb, Ta, Cr, Mo, W, Mn, Zn, Ga, In, P, lanthanides, and any combination thereof. Said promoter is present in a mass content of more than 0% by weight and less than 50% by weight, calculated on the basis of the total mass of the catalyst. Preferably, said promoter is present in a mass content larger than 0% by weight and lower than 25% by weight. Preferably, said promoter element is in ionic form, for example, as oxide or suboxide, in the catalyst.

According to a preferred embodiment, the promoter is an oxide selected from the list of stable oxides of elements from groups 3, 4, 5, 6, 7, 12, 13, 14, 15, and lanthanides of the periodic table, and any combination thereof.

Suitable methods for incorporating additional active metals other than ruthenium, cobalt, or combinations thereof, preferably ruthenium, as well as promoter elements, include techniques known in the art, such as impregnation, precipitation-deposition, ion exchange, electrochemical deposition, electrostatic adsorption, melt infiltration, or coprecipitation with suitable precursors, such as inorganic metal salts selected from the non-limiting list of nitrates, halides, carbonates, sulphates, and combinations thereof, organic salts selected from the non-limiting list of acetates, acetylacetonates, oxalates, ethoxylates, and combinations thereof, metal clusters and organometallic complexes.

Preferably, the weight loading of the active metal, in other words, the weight fraction of the total catalyst corresponding to the active metal in said catalyst, is in the range of 0.5-50% by weight, and more preferably in the range of 3-20% by weight.

Prior to its application in the method of the invention, be it unsupported or supported, the catalyst is typically subjected to thermal treatments in the presence of hydrogen or an alternative reducing agent to convert part or all of the active metal into its metallic state, in other words, the zero-valent state. In a preferred embodiment, said reduction treatment takes place at a temperature in the range of 373-873 K, and more preferably in the range 373-773 K in flow of a stream comprising hydrogen, either as a pure gas or in combination with an inert gas from the list of nitrogen, helium, argon, or combinations thereof. Said thermal treatment may be omitted in cases where exposure to the conditions of the reaction process, in other words, in the presence of synthesis gas as a reducing atmosphere, is sufficient to convert part or all of the active metal to its metallic state, *in situ,* in the reactor of the method of the invention.

The present invention will now be described in more detail by means of the following figures and experimental examples.

Throughout the description and the claims, the word "comprises" and its variants do not intend to exclude other technical features, additives, components or steps. For those skilled in the art, other objects, advantages and features of the invention may be partially deduced from both the description and the embodiment of the invention.

## FIGURES

**Figure 1:** Figure 1 shows the evolution of the relative abundance of products with alkyl chain lengths in the range of $C_{7-30}$, that is, corresponding to the fatty alkylamine range, along the Y axis, in arbitrary units, increasing from bottom to top, as a function of the chain growth probability parameter (dimensionless) according to an Anderson-Schulz-Flory (ASF) distribution, along the X axis, increasing from left to right. The line connecting the scatter data dots corresponds to the interpolated spline function.

## EXAMPLES

The following examples are given by way of illustration but are not intended to limit the scope of the invention.

The following methods have been used to determine the properties of the materials prepared and used in the examples:

(i) The specific surface area and pore volume of the support materials used for catalyst synthesis were determined by nitrogen adsorption. Nitrogen adsorption isotherms were recorded using the ASAP 2420 equipment (Micromeritics) at -196°C (77 K). Before analysis, about 200 mg of the sample (0.2-0.4 mm sieve fraction) were degassed at 673 K and $\sim 5\times 10^{-6}$ bar overnight. The specific surface area was determined by applying the Brunauer-Emmett-Teller (BET) equation in the relative pressure (P/P°) range of 0.05-0.3. The pore volume was determined from nitrogen uptake at a relative pressure $P/P^0=0.95$.

(ii) The elemental composition of the catalysts was determined by energy dispersive X-ray spectroscopy (EDS) in a ZEISS ULTRA-55 field emission scanning electron microscope (FE-SEM), equipped with an X-Max 80 - Oxford Instruments EDS detector. The powder samples were dispersed onto a pin stub coated by a double adhesive face carbon film. Next, areas of dispersed material of at least $5*10^4$ $\mu m^2$ were analysed using 20 kV accelerating voltage. The elemental composition was determined by integrating the K-series spectral lines of the X-ray emission spectra for elements with atomic number Z equal or lower than 30, and integrating the L-series spectral lines for elements with atomic number Z greater than 30.

### Catalyst synthesis according to the invention

#### *Ru/$SiO_2$ catalyst*

A ruthenium precursor solution was prepared by dissolving ruthenium (III) nitrosyl nitrate ($N_4O_{10}Ru$, Alfa Aesar,

Ru 31.3% min) in 0.1 M $HNO_3$ in deionised water. Specifically, 1.0 g of metal precursor was added to 2.0 mL of acid solution. A silica gel powder (S10020M - Silicycle) was used as a catalyst support. The metal was incorporated by incipient wetness impregnation. The $SiO_2$ support was first dried in a glass flask at 423 K for 10 h under dynamic vacuum (1.5 mbar). Next, a volume of ruthenium precursor solution equivalent to 90% of the pore volume of the $SiO_2$ support (0.72 $cm^3$/g) was brought in contact with the dry support, under magnetic stirring and static vacuum. After the impregnation, the material was kept under dynamic vacuum (1.5 mbar) for at least 3 hours, transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. After the thermal treatment, the tubular reactor was purged with a $N_2$ flow and the activated catalyst was transferred to a $N_2$-filled glove box ($O_2$< 1.0 ppm), $H_2O$< 0.1 ppm) for storage. The Ru content was 8.0% by weight.

***Ru/$Al_2O_3$ catalyst***

The Ru/$Al_2O_3$ catalyst was synthesised as detailed above for Ru/$SiO_2$, using in this case $\gamma$-$Al_2O_3$ as the support material. The $\gamma$-$Al_2O_3$ support was synthesised by calcination of a pseudo-boehmite precursor (DISPERAL 80, Sasol) at 823 K (heating ramp of 2 K/min from room temperature) for 5 h in a muffle oven under air atmosphere without forced convection. The support has a pore volume of 0.66 $cm^3$/g. Following incorporating Ru by incipient wetness impregnation, the solid was dried at room temperature for at least 3 h under dynamic vacuum (1.5 mbar), transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. The Ru content was 7.4% by weight.

***$WO_3$-Ru/$SiO_2$ catalyst***

The $WO_3$-Ru/$SiO_2$ catalyst was synthesised by incorporating tungsten oxide as a promoter on the Ru/$SiO_2$ catalyst. A tungsten precursor solution was prepared by dissolving ammonium metatungstate hydrate ($(NH_4)_6W_{12}O_{39}$-$xH_2O$, Alfa Aesar) in 0.1 M $HNO_3$ in deionised water. Specifically, 1.1 g of tungsten salt were dissolved in 10 mL of acid solution. The catalyst was synthesised as detailed above for Ru/$SiO_2$ using, in this case, the Ru/$SiO_2$ catalyst as the support material, considering a pore volume of 0.71 $cm^3$/g. Lastly, the solid was dried under dynamic vacuum (1.5 mbar) for at least 3 h at room temperature, transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. The contents of ruthenium and tungsten in the $WO_3$-Ru/$SiO_2$ catalyst were 8.2% by weight and 4.5% by weight, respectively.

***Ru/$PrO_x$-$Al_2O_3$ catalyst***

The Ru/$PrO_x$-$Al_2O_3$ catalyst was synthesised by incorporating praseodymium oxide as a promoter. First, praseodymium oxide was deposited on a $\gamma$-$Al_2O_3$ support material. A praseodymium solution was prepared by dissolving praseodymium(III) nitrate hexahydrate ($Pr(NO_3)_3$-$6H_2O$, Alfa Aesar, 99.99%) in deionised water. Specifically, 1.42 g of metal precursor were added to 2 mL of deionised water. Separately, the $\gamma$-$Al_2O_3$ support was synthesised by calcination of the pseudo-boehmite precursor (DISPERAL HP-14, Sasol) at 823 K (heating ramp of 2 K/min from room temperature) for 5 h in a muffle oven under air atmosphere without forced convection. The Pr solution was incorporated onto the $\gamma$-$Al_2O_3$ support by incipient wetness impregnation. The $\gamma$-$Al_2O_3$ support was first dried in a glass flask at 423 K overnight under dynamic vacuum (1.5 mbar). Next, a volume of praseodymium precursor solution equivalent to 90% of the pore volume of the support (0.74 $cm^3$/g) was brought in contact with the dry support under magnetic stirring and static vacuum. After the impregnation, the material was kept under dynamic vacuum (1.5 mbar) for at least 3 hours, transferred to a fixed-bed tubular quartz reactor mounted axially in an oven, and treated at 823 K for 4 h (heating ramp of 3 K/min from room temperature) under a synthetic air flow (200 mL/min) to decompose the Pr precursor into a supported oxide. The Pr content was 15.3% by weight, which corresponds to 5.5 Pr atoms/$nm^2$ of alumina specific surface area.

Next, ruthenium was incorporated into the $PrO_x$-$Al_2O_3$ support by impregnation. In a 100 mL glass flask, 0.61 g of support were added to 40 mL of deionised water and kept under vigorous magnetic stirring for 10 minutes. Next, 0.17 g of ruthenium(III) nitrosyl nitrate (N4O10Ru, Alfa Aesar, Ru 31.3% min) were added as the metal precursor, suspended in the aqueous solution, and kept under vigorous stirring for 30 minutes. Next, the solvent was evaporated to dryness in a rotary evaporator under vacuum (200 mbar) at 333 K, and the recovered solid was dried at 373 K for 2 h. Finally, the solid was dried at room temperature under dynamic vacuum (1.5 mbar) for at least 3 hours, transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. The Ru and Pr contents were 10.7% by weight and 14.1% by weight, respectively.

### *Ru/$ZrO_2$-$Al_2O_3$ catalyst*

The Ru/$ZrO_2$-Al2$O_3$ catalyst was synthesised by incorporating zirconium oxide as a promoter. First, zirconium oxide was deposited on a γ-$Al_2O_3$ support material. A zirconium solution was prepared by dissolving the zirconium(IV) isopropoxide complex ($Zr(OCH(CH_3)_2)_4$-$(CH_3)_2CHOH$, Sigma Aldrich, 99.9% trace metal basis) in isopropanol (Scharlab, EssentQ, 99.5% min). Specifically, 1.07 g of metal precursor were added to 2 mL of isopropanol. Separately, the γ-$Al_2O_3$ support was synthesised by calcination of the pseudo-boehmite precursor (DISPERAL HP-14, Sasol) at 823 K (heating ramp of 2 K/min from room temperature) for 5 h in a muffle oven under air atmosphere without forced convection. The Zr precursor was incorporated into the γ-$Al_2O_3$ support as has been detailed above for the $PrO_x$-$Al_2O_3$ material, obtaining the $ZrO_2$-$Al_2O_3$ material. The Zr content was 13.7% by weight, which corresponds to 5.7 Zr atoms/$nm^2$ of alumina specific surface area. Next, ruthenium was incorporated onto the $ZrO_2$-$Al_2O_3$ support by impregnation, as detailed above for Ru/$Al_2O_3$, considering a pore volume of 0.81 $cm^3$/g. Lastly, the solid was dried at room temperature under dynamic vacuum (1.5 mbar) for at least 3 hours, transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. The Ru and Zr contents were 9.6 and 8.3% by weight, respectively.

### *RuRh/$Al_2O_3$ catalyst*

A mixed ruthenium/rhodium precursor solution was prepared by dissolving ruthenium(III) nitrosyl nitrate ($N_4O_{10}Ru$, Alfa Aesar, Ru 31.3% min) as Ru metal precursor and rhodium(III) nitrate ($N_3O_9Rh$-x$H_2O$, Sigma Aldrich, 36% Rh basis) as Rh precursor in 0.1 M $HNO_3$ in deionised water. Specifically, 0.94 g of Ru metal precursor and 0.092 g of Rh metal precursor were added to 2.0 mL of acid solution to obtain an Ru:Rh molar ratio of 90:10. Separately, the γ-$Al_2O_3$ support was synthesised by calcination of a pseudo-boehmite precursor (DISPERAL 80, Sasol) at 823 K (heating ramp of 2 K/min from room temperature) for 5 h in a muffle oven. The support material had a pore volume of 0.66 $cm^3$/g. The mixed metal precursor solution was incorporated by incipient wetness impregnation and thermally decomposed as detailed above for Ru/$SiO_2$. The Ru and Rh contents were 6.7% by weight and 0.6% by weight of Rh, respectively.

### *RuCo/$Al_2O_3$ catalyst*

A mixed ruthenium/cobalt precursor solution was prepared by dissolving ruthenium(III) nitrosyl nitrate ($N_4O_{10}Ru$, Alfa Aesar, Ru 31.3% min) as Ru metal precursor and cobalt(II) nitrate hexahydrate ($Co(NO_3)_2 \cdot 6H_2O$, Sigma Aldrich, ACS reagent, 98%) as Co precursor in deionised water. Specifically, 0.23 g of Ru metal precursor and 0.025 g of Co metal precursor were added to 40 mL of deionised water to obtain an Ru:Co molar ratio of 90:10. Separately, the γ-$Al_2O_3$ support was synthesised by means of the calcination of a pseudo-boehmite precursor (DISPERAL 80, Sasol) at 823 K (heating ramp of 2 K/min from room temperature) for 5 h in a muffle oven. Next, the metals were incorporated onto the γ-$Al_2O_3$ support by impregnation, as detailed above for Ru/$PrO_x$-$Al_2O_3$. Lastly, the solid was dried at room temperature under dynamic vacuum (1.5 mbar) for at least 3 hours, transferred to a fixed-bed tubular quartz reactor mounted axially in an oven, and treated at 623 K for 3 h (heating ramp of 1 K/min from room temperature) under a $N_2/H_2$ flow (80% $N_2$, 20% $H_2$) to decompose the Ru precursor and convert the metal to its metallic state. The Ru and Co contents were 6.9 and 0.4% by weight, respectively.

### General method for catalysts testing according to the invention

In a general experimental method, the conversion process is carried out in a 316L stainless-steels, magnetically stirred, batch type reactor (ILSHIN), with an internal volume of 28 mL. Temperature reading is performed with a K-type thermocouple coated with a 316L stainless steel sheath inserted into the liquid reaction medium, while pressure reading of the gas phase in the headspace is taken with a digital pressure transducer (Sensys). Prior to the reaction tests, all liquid reagents were dried for 12 hours using an activated molecular sieve (5 Å, 3.2 mm mesh, Sigma Aldrich) and then stored in a $N_2$-filled glove box ($O_2$<1.0 ppm, $H_2O$<0.1 ppm). Also under air exclusion, the fine powder catalyst, the nitrogen-sourcing reactant, the solvent (when it is different from the nitrogen-sourcing reactant), and a magnetic stirring bar (L x D of 9 mm x 20 mm) coated with PTFE were added to a PTFE liner vessel and the latter capped and introduced into the autoclave body. Next, the autoclave was sealed and purged three times with a synthetic synthesis gas mixture containing CO/$H_2$/Ar (Ar used as internal standard for headspace gas chromatography analysis) in preset volumetric proportions, and then filled with the same synthesis gas mixture until a preset total pressure of P (bar) at room temperature. The reactor was introduced into a custom-made aluminium block mounted on a stirring/heating plate (Heidolph), the stirring rate was set to 800 rpm and the temperature inside the autoclave liner was ramped up to the preset reaction temperature at a heating rate of 3 K/min. After a selected reaction time, the reactor was cooled down to room temperature. The gas phase in the

headspace was injected into an Agilent 7890 gas chromatograph equipped with two analysis channels and using He as carrier gas. A first analysis channel is equipped with a packed column HP-PLOT-Q (30 m, ID of 0.32 mm, film thickness of 20.0 μ) and a packed column molecular sieve HP-MOLSIEVE (30 m, ID of 0.32 mm, film thickness of 12.0 μm), and a TCD detector for the analysis of permanent gases and carbon dioxide using Argon as internal standard. A second analysis channel is equipped with a DB 1 capillary column (60 m, ID of 0.32 mm, film thickness of 3 μm) and a FID detector for the analysis of hydrocarbons and nitrogen compounds. The liquid phase of the reaction product was recovered from the reactor and filtered to remove solid catalyst particles prior to analysis. Gas chromatographic analysis was performed on a Shimadzu GC-2010 Plus gas chromatograph equipped with a HP 5 capillary column (30 m, ID of 0.25 mm, film thickness of 0.25 μm) and a FID detector, which uses $H_2$ as carrier gas, using tetrahydrofuran (THF) as external standard. The reaction products were identified by mass spectrometry using a Shimadzu GCMS-QP2010 Ultra instrument.

**Example I**

In an example according to the present invention, 152 mg of $Ru/Al_2O_3$ were applied as catalyst, 0.71 mL of dipropylamine (Sigma Aldrich, 99%) were added as nitrogen-sourcing reactant, dissolved in 12 mL of 2,2,4-trimethylpentane (Sigma Aldrich, 99%) as solvent. The experiment was conducted according to the general catalytic testing method described above. The reaction time was 24 h.

**Example II**

In an example according to the present invention, 150 mg of $Ru/Al_2O_3$ were applied as catalyst, 0.60 mL of piperidine (Sigma Aldrich, 99%) were added as nitrogen-sourcing reactant, dissolved in 12 mL of 2,2,4-trimethylpentane (Sigma Aldrich, 99%) as solvent. The experiment was conducted according to the general catalytic testing method described above. The reaction time was 24 h.

**Example III**

In an example according to the present invention, 81 mg of $Ru/Al_2O_3$ were applied as catalyst and 5 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example IV**

In an example according to the present invention, 103 mg of $Ru/Al_2O_3$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example V**

In an example according to the present invention, 151 mg of $Ru/Al_2O_3$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example VI**

In an example according to the present invention, 152 mg of $Ru/SiO_2$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction temperature was 473 K and the reaction time was 24 hours.

**Example VII**

In an example according to the present invention, 151 mg of $WO_3$-$Ru/SiO_2$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The

reaction time was 24 h.

**Example VIII**

In an example according to the present invention, 150 mg of Ru/$PrO_x$-$Al_2O_3$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example IX**

In an example according to the present invention, 152 mg of Ru/$ZrO_2$-$Al_2O_3$ were applied as catalyst and 12 mL of N,N-dimethylformamide (Sigma Aldrich, 99.8%) to the reactor, acting both as nitrogen-sourcing reactant and solvent. The experiment was conducted according to the general catalytic testing method described above. The reaction time was 24 h.

**Example X**

In an example according to the present invention, 152 mg of RuRh/$Al_2O_3$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example XI**

In an example according to the present invention, 152 mg of RuCo/$Al_2O_3$ were applied as catalyst and 12 mL of N,N-dimethylformamide (Sigma Aldrich, 99.8%) were added to the reactor, acting both as nitrogen-sourcing reactant and as solvent. The experiment was conducted according to the general catalytic testing method described above. The reaction time was 24 h.

**Example XII**

This is a comparative example, not according to the present invention, in which, instead of the solid ruthenium catalysts of the invention, an organometallic ruthenium molecular catalyst has been applied. The catalyst is obtained by combining a Ru precursor and a quaternary onium salt as a ligand, in the reaction medium, as described in document US4543411A. Experimentally, 25 mg of $Ru_3(CO)_{12}$ (Sigma Aldrich, 99%) and 302 mg of tetrabutylphosphonium bromide (Sigma Aldrich, 98%) were applied as catalyst, 0.62 mL of diethylamine (Sigma Aldrich, 99,5%) were added as nitrogen-sourcing reactant and 12 mL of N,N-dimethylformamide (Sigma Aldrich, 99.8%) were added as solvent. The experiment was carried out according to the general catalytic testing method described above. The reaction time was 24 h.

**Table 1:** Summary of the product conversion, selectivities, and yields for the experimental examples according to the present invention (Examples I-XI) and the comparative example not according to the present invention (Example XII).

| Example No. | T[(a)] (K) | p(b) (bar) | $H_2$:CO (-) | CO:Metal[(c)] (-) | $X_{CO}$[(d)] (%) | Selectivity (C%)[(d)] | | | Yield to fatty amines $C_7$ - $C_{30}$ (mg $g_{Metal}^{-1}$ $h^{-1}$)[(f)] | α[(g)] (-) |
|---|---|---|---|---|---|---|---|---|---|---|
| | | | | | | $CO_2$ | Hydrocarbons[(e)] | Alkylamines | | |
| **I** | 433 | 39 | 1 | 70:1 | 67.2 | 1.3 | 16.5 | 56.7[(h)] | 64.7 | 0.86 |
| **II** | 433 | 41 | 1 | 80:1 | 45.3 | 3.2 | 15.9 | 17.3[(h)] | 33.5 | 0.86 |
| **III** | 413 | 43 | 1 | 240:1 | 6.9 | 10.3 | 10.6 | 79.1 | 26.0 | 0.85 |
| **IV** | 433 | 42 | 2 | 80:1 | 85.4 | 7.3 | 22.2 | 70.5 | 99.6 | 0.86 |
| **V** | 473 | 45 | 1 | 80:1 | 76.0 | 16.9 | 8.0 | 75.1 | 80.6 | 0.87 |
| **VI** | 473 | 45 | 1 | 80:1 | 73.8 | 9.2 | 18.1 | 72.7 | 41.2 | 0.85 |
| **VII** | 433 | 41 | 1 | 80:1 | 26.0 | 2.2 | 26.5 | 71.3 | 26.6 | 0.83 |

(continued)

| Example No. | T[a] (K) | p(b) (bar) | $H_2$:CO (-) | CO:Metal[c] (-) | $X_{CO}$[d] (%) | Selectivity (C%)[d] | | | Yield to fatty amines $C_7$ - $C_{30}$ (mg $g_{Metal}^{-1}$ $h^{-1}$)[f] | α[g] (-) |
|---|---|---|---|---|---|---|---|---|---|---|
| | | | | | | $CO_2$ | Hydrocarbons[e] | Alkylamines | | |
| **VIII** | 433 | 55 | 1 | 80:1 | 8.4 | 14.2 | 6.4 | 79.4 | 7.5 | 0.77 |
| **IX** | 433 | 66 | 1 | 100:1 | 28.5 | 8.2 | 7.6 | 84.2 | 67.8 | 0.82 |
| **X** | 433 | 40 | 1 | 90:1 | 52.7 | 8.9 | 11.5 | 79.6 | 91.5 | 0.87 |
| **XI** | 433 | 41 | 1 | 90:1 | 39.9 | 12.1 | 10.0 | 77.9 | 50.5 | 0.84 |
| **XII** | 433 | 40 | 1 | 70:1 | 5.1 | 10.2 | 15.9 | 17.5[h] | nd[i] | na[j] |

(a) Reaction temperature; (b) Reaction pressure, determined at the reaction temperature; (c) Molar ratio between CO in the synthesis gas feed and the active metal of the catalyst; (d) CO conversion; (d) Selectivity on a carbon molar basis; (e) Non-nitrogenated hydrocarbons (paraffins, olefins, etc.); (f) Based on the total mass of the active metal, i.e., Ru and its combinations with Co, Rh, or Ir; (g) chain growth probability parameter as determined from the analysis of the alkyl chain length ($C_n$) in alkylamine products according to an ASF distribution in the chain length range n=5-21; (h) the remaining products include other nitrogenated compounds such as N,N-alkylamides; (i) not detected; (j) not applicable.

As can be deduced from the results presented in Table 1, the method of the invention (Examples I-XI) leads to high alkylamine selectivity in a single conversion step. Alkylamine products are described by chain growth probability parameters for alkyl groups in the range of 0.80-0.90, particularly suitable for producing fatty amines. On the contrary, as exemplified in comparative example XII, a molecular ruthenium catalyst, which has been shown to be effective for the production of alkylamines from aliphatic olefins according to the method described in the document US4543411A, is not effective for producing alkylamines, and particularly fatty alkylamines, in the method of the invention, in which syngas is fed as the sole carbon source for the development of alkyl groups.

Although the present invention has been described in terms of preferred embodiments, it is understood that such description should not be construed as limiting the invention described herein. Upon reading the description, it will be immediately apparent to those with ordinary skill in the art, in view of the teachings of this invention, that various alterations and modifications can be made thereto. The appended claims are to be interpreted as encompassing all alterations and modifications falling within the spirit and scope of the present invention.

## Claims

**1.** A method for the direct synthesis of alkylamines, which comprises at least the following steps:

i) providing a reactor with

a. a synthesis gas feed, and
b. a liquid feed comprising a nitrogen-sourcing reactant selected from primary amines, acyclic secondary amines, cyclic secondary amines including non-N-substituted compounds piperidine, morpholine, pyrrolidine, pyrrole, imidazole, N,N-dialkylformamide compounds, N,N-cycloalkylformamide compounds, and any combination thereof,

ii) reacting the feed provided in i) over a solid catalyst comprising at least one active metal selected from the list of ruthenium, cobalt, or any combination thereof, either in unsupported form or dispersed on a support material, at a reaction temperature comprised between 373 and 523 degrees Kelvin and a total pressure comprised between 1 bar and 300 bar; and
iii) recovering alkylamine compounds from the reaction products of step ii).

**2.** The method according to claim 1, wherein the nitrogen-sourcing reactant is an N,N-dialkylformamide.

3. The method according to claim 2, wherein the nitrogen-sourcing reactant is N,N-dimethylformamide.

4. The method according to any of the preceding claims, wherein step b further comprises a solvent selected from aliphatic hydrocarbons, aromatic hydrocarbons, oxygenated compounds, water, nitrogen compounds, halogenated organic compounds, liquid organic salts, and any combination thereof.

5. The method according to any one of the preceding claims, wherein the solid catalyst further comprises, as an active metal, rhodium, iridium, or any combination thereof.

6. The method according to any of the preceding claims, wherein the solid catalyst is a supported catalyst in which the active metal is dispersed on a support material which is selected from the group of $SiO_2$, $Al_2O_3$, $TiO_2$, carbon, or any combination thereof.

7. The method according to claim 6, wherein the weight fraction of the total catalyst corresponding to the active metal is in the range of 0.5-50% by weight.

8. The method according to any one of the preceding claims, wherein the solid catalyst further comprises an oxide selected from the list of stable oxides of elements from groups 3, 4, 5, 6, 7, 12, 13, 14, 15 and lanthanides of the periodic table, and any combination thereof.

9. The method according to any one of the preceding claims, wherein the reaction is carried out in a stirred discontinuous reactor.

10. The method according to any of claims 1 to 8, **characterised in that** the reaction is carried out in a continuous reactor to which both the gas stream and the liquid stream are fed.

11. The method according to any one of the preceding claims, **characterised in that** the reaction temperature is in the range between 393 K and 453 K.

12. The method according to any one of the preceding claims, **characterised in that** the reaction pressure is in the range of 10 bar to 300 bar.

13. The method according to any one of the preceding claims, **characterised in that** the $H_2$/CO molar ratio in the synthesis gas feed is comprised between 0.1 and 10.

14. The method according to any one of the preceding claims, **characterised in that** the alkylamine products are tertiary fatty amines with molecular formula R-N-($B_1$,$B_2$), wherein the substituent group R is an n-alkyl group having between 7 and 30 carbon atoms, and wherein the substituent groups $B_1$ and $B_2$ are selected from the list of methyl, ethyl, propyl, or isopropyl.

15. The method according to claim 14, **characterised in that** the substituent groups $B_1$ and $B_2$ are methyl groups.

16. The method according to any one of claims 1 to 13, **characterised in that** the alkylamine products are tertiary fatty amines with molecular formula R-N-($B_1$,$B_2$), wherein the substituent group R is an n-alkyl group having between 7 and 30 carbon atoms, and wherein the substituent groups $B_1$ and $B_2$ are the two ends of a ring having four carbon atoms or a ring having five carbon atoms.

17. The method according to any one of claims 1 to 16, wherein the synthesis gas feed further comprises other compounds such as nitrogen, helium, argon, water, $C_1$-$C_4$ alkanes, or combinations thereof.

18. The method according to any one of the preceding claims, **characterised in that**, after recovering alkylamines from the reaction products, a stream containing reagents that have not been converted, solvent, unrecovered reaction products, or a combination thereof, is recirculated to the reactor.

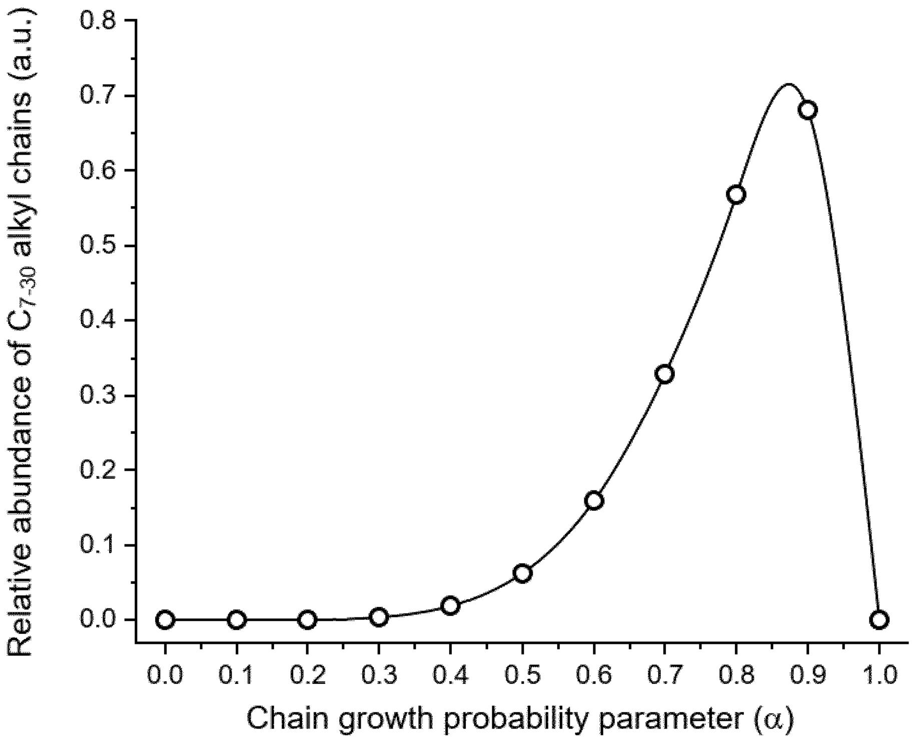
Relative abundance of $C_{7-30}$ alkyl chains (a.u.)
0.8
0.7
0.6
0.5
0.4
0.3
0.2
0.1
0.0
0.0 0.1 0.2 0.3 0.4 0.5 0.6 0.7 0.8 0.9 1.0
Chain growth probability parameter (α)

**FIG. 1**

# INTERNATIONAL SEARCH REPORT

International application No.
PCT/ES2023/070456

A. CLASSIFICATION OF SUBJECT MATTER

***C07B43/04*** (2006.01)
***C07C209/60*** (2006.01)
According to International Patent Classification (IPC) or to both national classification and IPC

B. FIELDS SEARCHED

Minimum documentation searched (classification system followed by classification symbols)
C07B, C07C

Documentation searched other than minimum documentation to the extent that such documents are included in the fields searched

Electronic data base consulted during the international search (name of data base and, where practicable, search terms used)

EPODOC, INVENES

C. DOCUMENTS CONSIDERED TO BE RELEVANT

| Category* | Citation of document, with indication, where appropriate, of the relevant passages | Relevant to claim No. |
|---|---|---|
| A | US 4543411 A (KNIFTON JOHN F ET AL.) 24/09/1985, column 6, line 58 - column 7, line 8; column 7, line 54 - column 8, line 24; claim 1, claim 9 | 1-18 |
| A | WO 2021225870 A1 (UNIV WASHINGTON STATE) 11/11/2021, paragraph [0011]; paragraph [0067]; claim 12 | 1-18 |
| A | US 4250115 A (IMAI TAMOTSU) 10/02/1981, claims 1-7 | 1-18 |
| A | US 4096150 A (BERTHOUX JEAN ET AL.) 20/06/1978, claim 1 | 1-18 |

☐ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 13/09/2023 | **(15/09/2023)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>J. Peces Aguado<br><br>Telephone No. 913496870 |

Form PCT/ISA/210 (second sheet) (July 2022)

## INTERNATIONAL SEARCH REPORT

Information on patent family members

International application No.
PCT/ES2023/070456

| Patent document cited in the search report | Publication date | Patent family member(s) | Publication date |
|---|---|---|---|
| US4543411 A | 24.09.1985 | NONE | |
| WO2021225870 A1 | 11.11.2021 | US2023144422 A1 | 11.05.2023 |
| US4250115 A | 10.02.1981 | NONE | |
| US4096150 A | 20.06.1978 | NL7317266 A | 24.06.1974 |
| | | JPS4988812 A | 24.08.1974 |
| | | JPS5238527B B2 | 29.09.1977 |
| | | IT1000323 B | 30.03.1976 |
| | | GB1408359 A | 01.10.1975 |
| | | FR2211002 A5 | 12.07.1974 |
| | | DE2363324 A1 | 11.07.1974 |
| | | DE2363324 B2 | 26.05.1976 |
| | | BE808841 A | 19.06.1974 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7642383 B2
- US 4138437 A
- US 5043453 A
- US 4250115 A
- US 4543411 A
- US 4096150 A
- WO 2005077884 A2
- DE 10321421 A1
- US 2518754 A
- US 2821537 A
- US 3726926 A
- WO 2009127942 A2
- WO 225870 A1


### Non-patent literature cited in the description


- **A. BAIKER ; J. KIJENSKI**. Catalytic Synthesis of Higher Aliphatic Amines from the Corresponding Alcohols. *Catal. Rev. -Sci. Eng.*, 1985, vol. 27 (4), 653-697
- **REPPE, W. ; VETTER, H.** Carbonylierung VI. Synthesen mit Metallcarbonylwasserstoffen. *Justus Liebigs Annalen der Chemie*, 1953, vol. 582, 133-161
- Fischer-Tropsch Synthesis. Catalysts, and Catalysis: Advances and Applications. CRC Press, 2016
- **T. SANGO et al.** *Applied Catalysis A: General*, 2015, vol. 502, 150-156
- Handbook of Heterogeneous Catalysis. Wiley-VCH Verlag GmbH & Co., 2008, vol. 1
- Synthesis of solid catalysts. Wiley-VCH Verlag GmbH & Co., 2009